# FASCICULE DE BREVET EUROPEEN

(11) **EP 2 020 236 B1**
(45) Date de publication et mention de la délivrance du brevet: **27.01.2016**
(21) Numéro de dépôt: 08168565.3
(22) Date de dépôt: 24.10.2000
(51) Int. Cl.: A61K 31/57, A61K 31/565

(54) **Méthode contraceptive à base d'un progestatif et d'un estrogéne**
Verfahren zur Empfängnisverhütung auf der Basis eines Progestativs und eines Oestrogens
Method of contraception based on a progestative and oestrogen

(30) Priorité: 25.10.1999 WO PCT/FR99/02587
(43) Date de publication de la demande: 04.02.2009
(62) Demande divisionnaire de: 00971488.2
(73) Titulaire: LABORATOIRE THERAMEX, 98000 Monaco (MC)
(72) Inventeur: Thomas, Jean-Louis, 94220, CHARENTON-LE-PONT (FR); Paris, Jacques, 06100, NICE (FR)
(74) Mandataire: Nevant, Marc

(56) Documents cités:
- FR-A- 2 754 179

## Description

Parmi les moyens contraceptifs les plus utilisés et les plus efficaces figurent les associations hormonales qui agissent par 3 mécanismes différents qui sont, par ordre d'importance :
- l'inhibition de la fonction gonadotrope qui freine la sécrétion par l'hypophyse de la FSH et de la LH et empêche de ce fait la maturation du follicule ovarien et la survenue du pic ovulatoire de LH indispensable à la ponte ovulaire ;
- les modifications de la sécrétion et des propriétés physico-chimiques de la glaire cervicale, qui la rendent imperméable aux spermatozoïdes ;
- l'inhibition du développement de la muqueuse utérine qui devient impropre à la nidation.

Jusqu'alors, dans les combinaisons estro-progestatives utilisées pour la contraception, l'inhibition de la fonction gonadotrope était due principalement à la fraction estrogénique constituée par un estrogène de synthèse : l'éthinyl-estradiol. Grâce à l'utilisation simultanée de dérivés de la 19-nor-testostérone, la fraction progestative renforce cette inhibition de l'ovulation, et assure en outre les effets contraceptifs périphériques sur la glaire cervicale et l'endomètre.

Cependant, l'utilisation des associations contraceptives estro-progestatives actuellement disponibles présente des inconvénients importants.
L'éthinyl-estradiol a un très fort impact sur les fonctions hépatiques ; ceci se traduit essentiellement par des troubles de la synthèse des facteurs de la coagulation et par des anomalies du profil plasmatique des lipides (BONNAR et al, 1987 ; MEADE, 1988 ; LINDBERG et al, 1989 ; VON SHOULTZ et al, 1989 ; DALY et BONNAR, 1990; BURKMAN, 1997 ; SPITZER, 1997). De ce fait, l'utilisation des contraceptifs estro-progestatifs est problématique chez les femmes à risque (femmes atteintes de troubles circulatoires, femmes en périménopause, femmes fumeuses...). Ceci est d'autant plus important que l'effet délétère de l'éthinyl-estradiol peut être encore augmenté par la fraction progestative du fait d'une activité androgénique résiduelle fréquemment présente (BONNAR, 1987 ; SABRA et BONNAR, 1983 ; BONNAR et al, 1987).

La fraction progestative des contraceptifs estro-progestatifs actuellement disponibles est le plus souvent constituée d'un dérivé de 19-nortestostérone qui a, comme l'éthinyl-estradiol, un impact négatif sur la fonction hépatique, le profil lipidique et les vaisseaux. Bien que cela n'ait pas été démontré de façon définitive, les dérivés 19-nor-testostérone les plus modernes, progestatifs dits de 3ème génération, sont suspects d'induire une augmentation des accidents thrombo-emboliques (O'BRIEN, 1999).

Pour échapper aux inconvénients de l'éthinyl-estradiol, les dérivés de 19-nortestostérone sont parfois utilisés seuls en contraception, selon 2 modalités différentes :
- soit à faibles doses, et dans ce cas l'action contraceptive est assurée par les seuls effets périphériques du progestatif ; en effet, l'inhibition de l'ovulation n'est pas constante car les faibles doses de progestatif permettent très souvent le développement des follicules ovariens et, dans certains cas, une augmentation de la sécrétion endogène d'estradiol ;
- soit à fortes doses, de façon à inhiber, à coup sûr, l'ovulation, mais au risque de créer une hypoestrogénie, ce qui limite leur utilisation chez les femmes jeunes.

En résumé, il apparaît très utile de disposer d'une association estro-progestative aussi efficace que celles qui sont actuellement disponibles mais qui soit dénuée de leurs effets secondaires néfastes.
Pour ce faire, il était facile de :
- remplacer l'éthinyl-estradiol (EE) par l'hormone sécrétée par l'ovaire, le 17-beta estradiol (E2) beaucoup moins toxique que EE (BUCKMAN et al, 1980 ; BERGINK et al, 1981 ; LINDBERG et al, 1989) mais faiblement antigonadotrope (HIRVONEN, 1995). Les tentatives ont été nombreuses mais aucune n'a abouti à un produit proposé aux femmes. En général, l'effet anti-ovulatoire était bien obtenu mais les nombreux échecs étaient dus, dans la plupart des cas, à un mauvais contrôle du cycle menstruel avec apparition de spottings et de saignements intermenstruels qui rendaient la méthode inacceptable.
Ainsi les associations d'estrogènes naturels avec le désogestrel (WENZL,1993 ; KIVINEN et SAURE, 1996 ; CSEMICZKY et al, 1996), avec l'acétate de cyprotérone (HIRVONEN et al, 1988 ; HIRVONEN et al, 1995), avec la norethistérone (ASTEDT et al, 1977 ; WORLD HEALTH ORGANIZATION, 1980 ; SERUP et al, 1981) se sont révélées être anticonceptionnelles mais les saignements intercurrents, les spottings et la mauvaise qualité des règles n'étaient pas acceptables. Pour certains, les raisons de ces échecs résidaient dans une stimulation estrogénique insuffisante du fait de la mauvaise biodisponibilité de l'estradiol ou de ses esters ; l'effet progestatif trop puissant conduisait à une inhibition partielle de la prolifération endométriale et donc à des saignements anarchiques (HIRVONEN et al, 1995 ; CSEMICSKY et al, 1996). Une seule association a donné des résultats satisfaisants en terme de contrôle du cycle menstruel ; il s'agit de l'association valérate d'estradiol et dienogest (OETTEL et al 1999 ; HOFFMAN et al, 1999). Selon ces auteurs, les résultats positifs seraient dus à une forte dissociation entre activité centrale (anti-ovulatoire) et activité périphérique (endométriale) au profit de cette dernière activité pour le dienogest. En résumé, l'ensemble des données publiées montre que le résultat dépend étroitement de l'effet antigonadotrope du progestatif, de la biodisponibilité de l'estradiol ou de ses dérivés dans la formulation utilisée et d'un rapport optimum entre les stimulations estrogène et progestative.
- remplacer le dérivé de 19-nortestostérone par un progestatif de synthèse fortement antigonadotrope et connu pour n'avoir pas d'impact sur la fonction hépatique, le métabolisme glucido-lipidique et les facteurs de coagulation.

### Effet contraceptif de l'association nomegestrol acétate / estradiol

L'objet de la présente invention est une nouvelle formulation contraceptive orale chez la femme en âge de procréer (jeune ou periménopausée) ; cette formulation repose sur la combinaison :
1. d'un progestatif de synthèse dénué de tout effet métabolique secondaire, le nomegestrol ou ses esters, dont l'effet antigonadotrope se trouve, de façon inattendue, potentialisé par l'estradiol ou par ses esters ;
2. de l'estradiol ou d'un de ses dérivés éthers ou esters pour compenser l'hypoestrogénie induite par le progestatif administré de façon prolongée durant le cycle ;
3. et l'utilisation d'un rapport pondéral optimum entre la fraction estrogénique et la fraction progestative pour assurer un bon contrôle du cycle menstruel.
La composante estrogénique fait appel à l'estradiol ou à un de ses esters comme par exemple le valérianate, le benzoate, l'énanthate..., les doses utilisées étant calculées en équivalent d'estradiol. Les doses s'échelonnent de 0,5 à 2 mg par jour. D'après les données de la littérature (HIRVONEN, 1995), une dose de 4 mg est nécessaire pour assurer l'inhibition de l'ovulation mais elles correspondent aux doses utilisées pour compenser les états d'hypoestrogénie. Par exemple, chez la femme ménopausée, la dose recommandée pour compenser les états d'hypoestrogénie est de 1,5 mg environ.
La composante progestative fait appel à l'acétate de nomegestrol. La fourchette de doses est comprise entre 0,625 et 1,25 mg par jour. A ces doses très faibles, l'acétate de nomegestrol associé à l'estradiol inhibe l'ovulation ainsi que la maturation folliculaire dans 100 % des cas lorsque les deux principes actifs sont administrés ensemble du 1^{er} au 21^{ème} jour du cycle, avec des fréquences acceptables d'hémorragie de privation et de saignements intercurrents.

La fourchette du rapport pondéral des doses d'estradiol sur les doses d'acétate de nomegestrol s'étend environ de 0,5 à 5 et ce rapport est compris de préférence entre 1 et 3 environ.

L'association de l'acétate de nomegestrol et de l'estradiol est administrée quotidiennement, à la même dose, à partir du 1^{er} jour du cycle, pendant une durée pouvant aller de 21 à 28 jours. Ensuite, les femmes reçoivent quotidiennement un comprimé placebo pendant le laps de temps nécessaire pour terminer le cycle de 28 jours (de 0 à 7 jours).

L'acétate de nomegestrol est un progestatif puissant, actif par voie orale et qui possède un profil pharmacologique original :
- comme les dérivés de 19-nortestosterone, l'acétate de nomegestrol est porteur d'une forte activité antigonadotrope mais à l'inverse des premiers, il ne manifeste aucune activité androgénique et estrogénique résiduelle et il a une forte activité anti-estrogénique.
- comme les dérivés de la 17alpha-hydroxyprogesterone, il présente un profil pharmacologique pur, mais à l'inverse de ceux-ci, il a un puissant effet antigonadotrope.
Il appartient à la catégorie des progestatifs qualifiés d'hybrides (OETTEL et al, 1999) qui ne sont pas porteurs d'effets métaboliques délétères du fait de l'absence de fonction 17α-ethinyle et qui combinent les avantages des dérivés de progestérone avec ceux des dérivés plus modernes de la 19-nortestosterone.
Un essai clinique similaire à l'essai de KAUFMANN a permis de montrer que la transformation endométriale est obtenue avec une dose quotidienne de 1 mg d'acétate de nomegestrol, soit 10 mg pour l'ensemble du cycle. Il avait été montré précédemment (BAZIN et al, 1987) que l'inhibition de l'ovulation et l'absence de développement folliculaire étaient obtenues chez la femme avec une dose quotidienne de 2.5 mg d'acétate de nomegestrol. Le rapport activité inhibitrice de l'ovulation chez la femme (en mg/jour) sur activité de luteinisation endométriale (en mg/cycle) tel que défini par NEUMANN (1977) est donc de l'ordre de 0,2, c'est-à-dire voisin de ceux de l'acétate de cyprotérone et de l'acétate de chlormadinone ; il signe une forte activité centrale (OETTEL et al, 1999). En ce sens, il se distingue nettement du dienogest dont l'activité est déséquilibrée au profit de l'activité périphérique. En conséquence, les résultats observés avec une association contraceptive valérate d'estradiol / dienogest ne suggèrent en rien et ne rendent pas évidents ceux observés avec l'association estradiol / acétate de nomegestrol selon l'invention.

L'étude du pouvoir anti-ovulatoire de l'association acétate de nomegestrol / estradiol montre une potentialisation inattendue des effets antigonadotropes de l'acétate de nomegestrol par l'estradiol puisque l'inhibition de l'ovulation et du développement folliculaire sont obtenues avec une dose faible, voisine de 0.625 mg. Ce résultat ne peut provenir d'un effet antigonadotrope de l'estradiol ni même d'une addition d'effets entre les deux principes actifs dans la mesure où les doses d'estradiol utilisées sont très inférieures aux doses connues pour inhiber l'ovulation (HIRVONEN et al, 1995). En conséquence, cette constatation inattendue est le signe d'une réelle innovation, car elle permet l'utilisation de doses moindres de progestatif et donc une tolérance meilleure ; elle se distingue de l'objet du brevet français 2.754.179 appartenant à la société Demanderesse, dans lequel la fourchette de doses d'acétate de nomegestrol pouvait aller de 1,5 à 5 mg.
La présente invention concerne donc un agent estro-progestatif administré de façon monophasique à partir du 1^{er} jour du cycle pendant 21 à 28 jours. Elle se distingue des revendications de nombreux brevets qui décrivent l'association d'estradiol ou d'un ester d'estradiol administrée selon des modalités polyphasiques avec des doses modifiées de l'estrogène et/ou du progestatif d'une phase à l'autre et, quelquefois même, changement du progestatif d'une phase à l'autre. On citera à cet égard à titre d'exemple les brevets EP 770338, WO 9741868, WO 9909993, WO 9835682, WO US9817288, WO 9602486, WO 9707074, WO 9707083, WO 9707084, WO 9707085, WO 9707089, WO 9712785, WO 9712785, WO 9712786, WO 9712787, WO 9712788, WO 9712789, WO 23228, WO 9741868, WO 9913882, EP 491438, EP 491415, WO 9004330, EP 3092263, US 4628051, EP 0911029 A2, EP0770388 A1 et DE 3229612, ainsi que les publications de HIRVONEN et al (1988, 1995) qui décrivent une méthode contraceptive biphasique avec l'association valérate d'estradiol / acétate de cyproterone ou celle de HOFFMANN et al (1998) qui décrit une méthode contraceptive biphasique avec l'association valérate d'estradiol / dienogest.

La présente invention inclut une méthode de contraception associant le 17β-estradiol ou un de ses esters et le nomegestrol ou un de ses esters, préférentiellement l'acétate de nomegestrol. Cette méthode de contraception est innovante par rapport aux brevets et aux publications consacrés à des associations estro-progestatives d'estradiol (ou un de ses esters) et d'un progestatif administrées de façon monophasique, car l'ensemble de la littérature montre que le résultat clinique global est totalement dépendant de la nature du progestatif utilisé, de son profil pharmacologique, de ses effets sur l'axe hypothalamo-hypophysaire du rapport pouvoir "central"/pouvoir "périphérique" et du rapport d'activité estrogénique et progestative. Pour ces raisons, les méthodes de contraception monophasiques décrites dans des brevets, comme par exemple WO 95/17194, WO 9912531 et EP 0253607 et des publications comme par exemple celles qui traitent d'associations norethisterone / estradiol (ASTEDT et al, 1977 ; Task force on oral contraception, 1980 ; SERUP et al, 1981), celles qui traitent d'associations desogestrel / estradiol (WENZL et al, 1993 ; CSEMICSKY et al, 1996) ou d'associations de dienogest et d'estradiol (HOFFMANN et al, 1998) ne peuvent s'appliquer à l'association de l'acétate de nomegestrol / estradiol dans la mesure où elles ne sont validées que pour l'estrogène et pour le progestatif revendiqués. Il s'y ajoute le fait que la potentialisation constatée entre l'estradiol et l'acétate de nomegestrol rend inutile toute extrapolation de doses à partir du profil pharmacologique. De plus l'acétate de nomegestrol n'est jamais cité comme progestatif pouvant être utilisé. Les brevets EP 309263 et WO 9004330 citent la possibilité d'utiliser la 17alpha-19-nor progestérone et ses esters mais il faut relever d'une part que l'acétate de nomegestrol n'est pas un ester de 17alpha-19-norprogestérone, et que d'autre part les esters de 17alpha-19-norprogestérone sont porteurs de propriétés antidiurétiques qui les rendent impropres à l'utilisation chez la femme (PARIS et al, 1987).

Une composition préférée sera celle qui contient 0,625 mg d'acétate de nomégestrol et 1 mg d'estradiol ou 0,625 mg d'acétate de nomégestrol et 1,5 mg d'estradiol ou bien encore 0,625 mg d'acétate de nomégestrol et 2 mg d'estradiol.

Les compositions pharmaceutiques selon l'invention sont celles qui conviennent pour la voie digestive, notamment sous forme de comprimés nus ou pelliculés, de dragées, de gélules, de capsules, de pilules, de cachets ou des poudres aromatisées ou non. Elles contiennent un agent diluant et/ou une substance de charge et/ou un adjuvant de compression et/ou un agent lubrifiant et/ou un agent d'éclatement. Comme agent de pelliculage, on pourra citer l'hydroxypropylméthylcellulose (Hypromellose) ou l'acétophtalate de cellulose.

Comme agent liant, on pourra utiliser la polyvinylpyrolidone, la carboxyméthylcellulose, la carboxyméthylcellulose réticulée, la cellulose microcristalline, l'éthylcellulose, l'hydroxyéthylcellulose ou un amidon modifié chimiquement ou non. Comme substance de charge on pourra citer le carbonate de calcium, le carbonate de magnésium, le phosphate de magnésium, des argiles, des zéolites, de la terre d'infusoires, etc. Comme adjuvant de compression on pourra citer le lactose ou le sucre en poudre. Comme agent lubrifiant on pourra citer le talc, le stéarate de calcium, le stéarate de magnésium, la silice colloïdale. Comme agent d'éclatement, on pourra citer le mannitol, le carboxyméthylamidon du la polyvinylpolypyrolidone.

D'une manière générale, le poids des compositions selon l'invention s'échelonne entre 40 et 100 mg et la composition contient de 80 à 99 % de diluants et d'excipients pour de 1 à 20 % de principes actifs.
L'acétate de nomégestrol et l'estradiol peuvent être administrés simultanément combinés dans une seule formulation ou au contraire présentés sous deux formes pharmaceutiques à ingérer successivement ou simultanément.
La posologie journalière sera de 1 à 2 prises et la durée du traitement s'exercera pendant la totalité du mois. Au total, la dose moyenne mensuelle d'acétate de nomégestrol s'échelonnera de 8 mg à 75 mg. Les doses sont parfaitement tolérées.

### EXEMPLE 1 : exemples de formulations

L'association d'acétate de nomegestrol et d'estradiol est présentée sous forme de comprimés nus ou pelliculés.
Dans ces compositions, l'estradiol est introduit avantageusement dans le mélange final sous forme de prémélange contenant de 2 à 5 % d'estradiol dans de la povidone (10 à 25%) et du lactose (qsp 100%) comme par exemple :

| FORMULATIONS | EN MG/1 COMPRIME | EN % |
|---|---|---|
| Estradiol | 1,00 | 2,50 |
| Povidone | 6,00 | 15,00 |
| Lactose | 33,00 | 82,50 |
| Alcool isopropylique | Environ 6,14 | Environ 15,35 |
| H₂O déminéralisée | Environ 1,06 | Environ 2,67 |
| **TOTAL EN SEC** | **40,00** | **100,00** |

Ce prémélange est introduit dans le mélange final pour obtenir un comprimé par compression directe.
Les comprimés terminés, nus, pèsent en général de 60 à 90 mg et ont la formule globale suivante :

### FORMULATION DES COMPRIMES NUS

| **Composition** | |
|---|---|
| | **en mg/par comprimé** |
| - Prémélange d'estradiol qsp | 0,5 à 1,5 |
| - Acétate de nomegestrol | 0,300 à 2,500 |
| - Silice colloïdale | 0,300 à 1,500 |
| - Crospovidone | 2,500 à 5,000 |
| - Lactose | 4,000 à 40,000 |
| - Cellulose | 6,000 à 40,000 |
| - Acide stéarique | 0,900 à 3,00 |
| - Talc | 0,450 à 1,500 mg |

A titre d'exemple, on pourra citer les comprimés pesant 90 mg, de formule suivante :

### Exemples de formulations (FU = formulation unitaire) comprimés de 90 mg

| **FORMULATION*** | **FU mg/1 cp 90 mg** | **FU %** |
|---|---|---|
| Prémélange d'estradiol à 2,5 % | 40,000 | 44,45 |
| Acétate de nomegestrol | 0,300 | 0,33 |
| Silice colloïdale (Aérosil 200) | 0,495 | 0,55 |
| Crospovidone (Polyplasdone XL) | 3,240 | 3,60 |
| Lactose | 26,000 | 28,89 |
| Cellulose microcristalline (Avicel PH 101) | 17,265 | 19,18 |
| Acide stéarique AC68/50VG | 1,800 | 2,00 |
| Talc | 0,900 | 1,00 |
| **TOTAL** | **90,000** | **100,00** |

| **FORMULATION*** | **FU mg/1 cp 90 mg** | **FU %** |
|---|---|---|
| Prémélange d'estradiol à 2,5 % | 40,000 | 44,45 |
| Acétate de nomegestrol | 2,500 | 2,77 |
| Silice colloïdale (Aérosil 200) | 0,495 | 0,55 |
| Crospovidone (Polyplasdone XL) | 3,240 | 3,60 |
| Lactose | 24,900 | 27,67 |
| Cellulose microcristalline (Avicel PH 101) | 16,165 | 17,96 |
| Acide stéarique AC68/50VG | 1,800 | 2,00 |
| Talc | 0,900 | 1,00 |
| **TOTAL** | **90,000** | **100,00** |

| **FORMULATION*** | **FU mg/1 cp 90 mg** | **FU %** |
|---|---|---|
| Prémélange d'estradiol à 2,5 % | 60,000 | 66,67 |
| Acétate de nomegestrol | 0,300 | 2,77 |
| Silice colloïdale (Aérosil 200) | 0,495 | 0,55 |
| Crospovidone (Polyplasdone XL) | 3,240 | 3,60 |
| Lactose | 12,215 | 8,91 |
| Cellulose microcristalline (Avicel PH 101) | 13,050 | 14,50 |
| Acide stéarique AC68/50VG | 1,800 | 2,00 |
| Talc | 0,900 | 1,00 |
| **TOTAL** | **90,000** | **100,00** |

| **FORMULATION** | **FU mg**/**1 cp 90 mg** | **FU %** |
|---|---|---|
| Prémélange d'estradiol à 2,5 % | 60,000 | 66,67 |
| Acétate de nomegestrol | 0,625 | 0,69 |
| Kollidon 25 | 9,000 | 10,00 |
| Silice colloïdale (Aérosil 200) | 0,495 | 0,55 |
| Crospovidone (Polyplasdone XL) | 3,240 | 3,60 |
| Cellulose microcristalline (Avicel PH 101) | 13,050 | 14,50 |
| Acide stéarique AC68/50VG | 1,800 | 2,00 |
| Talc | 0,900 | 1,00 |
| Lactose | 0,890 | 0,99 |
| **TOTAL** | **90,000** | **100,00** |

| | | |
|---|---|---|
| * hors invention | | |

On peut réaliser également des comprimés nus pesant 60 mg de formule suivante :

### Exemples de formulations (FU = formulation unitaire) comprimés de 60 mg

| **FORMULATION*** | **FU mg/1 cp 60 mg** | **FU %** |
|---|---|---|
| Prémélange d'estradiol à 4,0 % | 25,000 | 41,67 |
| Acétate de nomegestrol | 0,300 | 0,50 |
| Silice colloïdale (Aérosil 200) | 0,324 | 0,54 |
| Crospovidone (Polyplasdone XL) | 3,000 | 5,00 |
| Lactose | 16,076 | 26,79 |
| Cellulose microcristalline (Avicel PH 101) | 13,500 | 22,50 |
| Acide stéarique AC68/50VG | 1,200 | 2,00 |
| Talc | 0,600 | 1,00 |
| **TOTAL** | **60,000** | **100,00** |

| **FORMULATION*** | **FU mg/1 cp 60 mg** | **FU %** |
|---|---|---|
| Prémélange d'estradiol à 4,0 % | 25,000 | 41,67 |
| Acétate de nomegestrol | 2,500 | 4,17 |
| Silice colloïdale (Aérosil 200) | 0,324 | 0,54 |
| Crospovidone (Polyplasdone XL) | 3,000 | 5,00 |
| Lactose | 14,976 | 24,96 |
| Cellulose microcristalline (Avicel PH 101) | 12,400 | 20,66 |
| Acide stéarique AC68/50VG | 1,200 | 2,00 |
| Talc | 0,600 | 1,00 |
| **TOTAL** | **60,000** | **100,00** |

| **FORMULATION*** | **FU mg/1 cp 60 mg** | **FU %** |
|---|---|---|
| Prémélange d'estradiol à 4,0 % | 37,500 | 62,50 |
| Acétate de nomegestrol | 0,300 | 4,17 |
| Silice colloïdale (Aérosil 200) | 0,324 | 0,54 |
| Crospovidone (Polyplasdone XL) | 3,000 | 5,00 |
| Lactose | 7,076 | 16,08 |
| Cellulose microcristalline (Avicel PH 101) | 10,000 | 8,71 |
| Acide stéarique AC68/50VG | 1,200 | 2,00 |
| Talc | 0,600 | 1,00 |
| **TOTAL** | **60,000** | **100,00** |

| **FORMULATION*** | **FU mg/1 cp 60 mg** | **FU %** |
|---|---|---|
| Prémélange d'estradiol à 4,0 % | 25,000 | 41,67 |
| Acétate de nomegestrol | 2,500 | 4,17 |
| Silice colloïdale (Aérosil 200) | 0,324 | 0,54 |
| Crospovidone (Polyplasdone XL) | 3,000 | 5,00 |
| Lactose | 14,976 | 24,96 |
| Cellulose microcristalline (Avicel PH 101) | 12,400 | 20,66 |
| Acide stéarique AC68/50VG | 1,200 | 2,00 |
| Talc | 0,600 | 1,00 |
| **TOTAL** | **60,000** | **100,00** |

| **FORMULATION** | **FU mg/1 cp 60 mg** | **FU %** |
|---|---|---|
| Prémélange d'estradiol à 4 % | 37,500 | 62,50 |
| Acétate de nomegestrol | 0,625 | 1,04 |
| Kollidon 25 | 7,000 | 11,67 |
| Silice colloïdale (Aérosil 200) | 0,324 | 0,54 |
| Crospovidone (Polyplasdone XL) | 3,000 | 5,00 |
| Cellulose microcristalline (Avicel PH 101) | 8,213 | 13,69 |
| Acide stéarique AC68/50VG | 1,200 | 2,00 |
| Talc | 0,600 | 1,00 |
| Lactose | 1,538 | 2,56 |
| **TOTAL** | **60,000** | **100,00** |

| | | |
|---|---|---|
| * hors invention | | |

Ces comprimés peuvent être pelliculés avec par exemple :
- des agents de pelliculage à base d'alcool polyvinylique de type OPADRY PVA "barrière humidité" (alcool polyvinylique, dioxyde de titane, talc purifié, lécithine, gomme xanthane, pigments, laques),
   ou
- des agents de pelliculage à base de cellulose de type SEPIFILM L.P. [H.P.M.C. (hydroxypropylméthylcellulose)], cellulose microcristalline, acide stéarique, pigments, laques).

### EXEMPLE II : potentialisation de l'effet antigonadotrope de l'acétate de nomegestrol par l'estradiol

L'action anti-ovulatoire de la combinaison estradiol-acétate de nomégestrol a été évaluée dans un essai randomisé en double aveugle, chez 38 femmes en période d'activité ovarienne, volontaires, en bonne santé, âgées de 18 à 35 ans, dont on avait préalablement vérifié par un dosage de la progestérone plasmatique et l'établissement d'une courbe de température qu'elles avaient des cycles menstruels ovulatoires.

Les femmes ont été suivies pendant deux cycles consécutifs : le premier était un cycle témoin sans traitement ; au cours du cycle suivant (cycle sous traitement), elles ont reçu un traitement hormonal administré per os quotidiennement du 1^{er} au 21^{ème} jour du cycle.
Selon la randomisation :
- 9 femmes ont reçu 1,5 mg d'estradiol + 0,625 mg d'acétate de nomégestrol (groupe A),
- 10 autres, 1,5 mg d'estradiol + 1,25 mg d'acétate de nomégestrol (groupe B),
- les 10 autres, 1,5 mg d'estradiol + 2,5 mg d'acétate de nomégestrol (groupe C),
- et les 9 autres, étaient traitées par l'acétate de nomégestrol seul à la dose de 2,5 mg (groupe D).

Au cours du cycle témoin, les paramètres hormonaux n'étaient pas significativement différents dans les quatre groupes.
Le *tableau 1* indique pour chaque paramètre hormonal les concentrations moyennes observées au cours des 21 jours de traitement.

Chez toutes les femmes et quelles que soient les doses administrées, les cycles sous traitement ont tous été anovulatoires, avec une disparition du pic medio-cyclique de LH et un taux de progestérone plasmatique inférieur à 1 ng/ml.

La comparaison des paramètres hormonaux dans les groupes C et D a permis de montrer que l'association de l'estradiol à l'acétate de nomégestrol avait non seulement augmenté significativement les taux plasmatiques d'estradiol, mais avait également renforcé l'effet antigonadotrope du progestatif. En effet, en présence d'estradiol, les taux de LH et de FSH ont été trouvés statistiquement inférieurs à ceux observés en administrant l'acétate de nomégestrol seul.

Lorsque l'acétate de nomégestrol est associé à l'estradiol, il exerce des effets antigonadotropes même aux faibles doses (0,625 et 1,25 mg), puisque les paramètres hormonaux n'ont pas été trouvés significativement différents dans les groupes A, B et C.
Cet effet synergique de l'estradiol est confirmé par la comparaison des résultats de cette étude à ceux d'un autre essai clinique réalisé selon la même méthodologie mais avec le progestatif seul. Cette confrontation montre en effet qu'à la dose de 1,25 mg d'acétate de nomegestrol, l'addition de l'estradiol n'a pas d'influence notable sur les taux plasmatiques de la progestérone et des gonadotrophines (LH et FSH). En revanche, l'addition d'estradiol abaisse d'environ 300 % le taux plasmatique d'estradiol dosé 24 heures après la prise médicamenteuse, paramètre qui est un bon reflet de la sécrétion endogène de l'ovaire (*tableau II*).

On sait que l'acétate de nomégestrol donné seul à raison de 1,25 mg par jour abolit l'ovulation et empêche la formation du corps jaune, tout en entraînant une élévation de l'estradiol plasmatique, ce qui témoigne d'un développement folliculaire sans ovulation, comme on le rencontre avec la micropilule progestative.

Les études ont donc montré que l'addition d'une dose d'estradiol, insuffisante pour bloquer l'ovulation à elle seule, renforce les effets anti-ovulatoires du progestatif et inhibe également la folliculogénèse, et maintient des taux d'estradiol nettement inférieurs à 100 pg/ml à distance de la prise médicamenteuse. Lorsqu'on l'associe avec l'estradiol, il est possible d'observer des effets anti-ovulatoires avec des doses d'acétate de nomégestrol plus faibles que celles initialement utilisées; ceci confirme, dans la nouvelle étude, les résultats obtenus avec 0,625 mg d'acétate de nomegestrol (NOMAC) par jour, associés à l'estradiol.

Dans cette étude, le relevé des saignements génitaux a permis d'évaluer l'influence de l'association estradiol / acétate de nomegestrol sur le cycle. Sur l'ensemble des femmes traitées par la combinaison estroprogestative, on a ainsi constaté que la durée du cycle n'excédait pas 1 mois chez 50% des cas, que les spottings étaient totalement absents chez une femme sur deux et que l'hémorragie de privation après arrêt du traitement était en moyenne de 5,4 jours et ne dépassait pas 7 jours chez 86 % des femmes. Ces données ne différaient pas entre les groupes. S'agissant du premier cycle de traitement, elles traduisent une tolérance satisfaisante ; il en effet connu que la qualité des cycles obtenus avec ce type d'association s'améliore après quelques cycles de traitement.

**Tableau I : Concentrations plasmatiques moyennes (m ± sem) des gonadotrophines (LH et FSH) et des stéroïdes ovariens (estradiol et progestérone) au cours d'un cycle sous traitement par 3 associations estradiol/acétate de nomégestrol (E2/NOMAC). Comparaison au traitement par acétate de nomégestrol seul.**

| Paramètre hormonal | GROUPE A (n=9) 1,5 mg E2 | GROUPE B (n=10) 1,5 mg E2 | GROUPE C (n=10) 1,5 mg E2 | GROUPE D (n=9) | p (ANOVA) | |
|---|---|---|---|---|---|---|
| | + | + | + | | | |
| | 0,625mg NOMAC | 1,25 mg NOMAC | 2,5 mg NOMAC | 2,5 mg NOMAC | | |
| | | | | | Comparaison A,B,C | Comparaison C et D |
| LH (mUI/ml) | 4,1 ± 0,51 | 3,0 ± 0,51 | 2,7 ± 0,49 | 5,6 ± 0,62 | 0,135 | 0,002 |
| FSH (mUI/ml) | 6,2 ± 0,42 | 6,6 ± 0,52 | 5,4 ± 0,75 | 7,6 ± 0,28 | 0,318 | 0,019 |
| Progestérone ng/ml | 0,11 ± 0,031 | 0,07 ± 0,024 | 0,03 ± 0,009 | 0,07 ± 0,014 | 0,068 | 0,056 |
| Estradiol pg/ml | 62,0 ± 7,90 | 57,6 ± 4,53 | 47,2 ± 5,61 | 31,9 ± 3,91 | 0,225 | 0,043 |

**Tableau II : Concentrations moyennes (m ± sem) des gonadotrophines (LH et FSH) et de l'estradiol dans le plasma avec 1,25 mg d'acétate de nomégestrol associés ou non à l'estradiol**

| Paramètre hormonal | Cycle | NOMAC 1,25 mg (n=3)¹ | NOMAC 1,25 mg + E2 1,5 mg (n=10)² |
|---|---|---|---|
| LH (mUI/ml) | Témoin | 4,5 (4,0-5,0) | 7,1 ± 0,82 |
| | Traité | 3,1 (2,6-3,7) | 3,0 ± 0,51 |
| FSH (mUI/ml) | Témoin | 4,3 (4,0-4,5) | 6,6 ± 0,28 |
| | Traité | 3,3 (2,5-4,2) | 6,9 ± 0,48 |
| Estradiol pg/ml | Témoin | 112,0 (64,8-203,8) | 132,9 ± 10,57 |
| | Traité | 158,8 (99,5-201,7) | 47,2 ± 5,61 |

| | | | |
|---|---|---|---|
| E2 = estradiol ; NOMAC = acétate de nomégestrol ¹ = m (étendue) ; ² = m ± sem | | | |

### EXEMPLE III : effet de l'association acétate de nomegestrol / estradiol sur l'endomètre

Une étude a été conduite pour tester les effets sur l'endomètre de plusieurs doses d'acétate de nomégestrol associées à une dose d'estradiol oral équivalente à 1,5 mg.

Au cours de cette étude, 179 femmes ménopausées depuis au moins 3 ans ont reçu quotidiennement en continu 2 mg de valérate d'estradiol combinés avec quatre doses différentes d'acétate de nomégestrol : 5 mg (n=47), 2,5 mg (n=42), 1,25 mg (n=43) et 0,625 mg (n=47).

L'impact de ces quatre associations sur l'endomètre a été évalué en mesurant l'épaisseur de l'endomètre par échographie endovaginale et en réalisant une biopsie de l'endomètre avant et en fin de traitement.

Le *tableau IV* indique les résultats de l'examen échographique. En fin de traitement, l'épaisseur moyenne de l'endomètre reste inférieure ou voisine de 4 mm. L'augmentation de l'épaisseur sous traitement est de 0,39 mm en moyenne avec la plus faible dose d'acétate de nomégestrol (0,625 mg/j). Elle augmente légèrement avec la dose mais reste inférieure à 1,5 mm avec 2,5 mg/j.

Les biopsies examinées en fin d'étude (*tableau V*) n'ont révélé aucun aspect prolifératif ou hyperplasique de la muqueuse utérine après 6 mois de traitement. C'est avec les plus faibles doses d'acétate de nomégestrol qu'on a observé le plus grand nombre d'endomètres atrophiques.

Ces résultats indiquent que de faibles doses d'acétate de nomégestrol administrées en continu avec l'estradiol sont capables d'imprégner suffisamment l'endomètre et d'empêcher à terme la croissance de la muqueuse utérine.

**Tableau III : Epaisseur endométriale après 6 mois de traitement par plusieurs associations combinées continues à base d'estradiol (2 mg de valérate d'estradiol) et d'acétate de nomégestrol (NOMAC) à plusieurs doses**

| | | | | |
|---|---|---|---|---|
| Doses de NOMAC (mg/j) | 0,625 | 1,25(n = 33) | 2,5 | 5 |
| | (n = 35) | | (n = 34) | (n = 41) |
| Epaisseur moyenne en fin de traitement (mm) | 3,18 | 4,05 | 3,93(2,10) | 3,83 |
| | (1,65) | (3,75) | | (2,72) |
| Augmentation moyenne de l'épaisseur sous traitement (mm) | 0,39 | 1,12 | 1,36 | 1,57 |
| | (1,67) | (3,67) | (1,54) | (2,39) |

| | | | | |
|---|---|---|---|---|
| ( ) = écart standard | | | | |

**Tableau IV : Aspect histologique de l'endomètre après 6 mois de traitement par plusieurs associations combinées continues à base d'estradiol (2 mg de valérate d'estradiol) et d'acétate de nomégestrol (NOMAC) à plusieurs doses**

| | | | | |
|---|---|---|---|---|
| Doses de NOMAC (mg/j) | 0,625 | 1,25 | 2,5 | 5 |
| | (n = 32) | (n = 33) | (n = 34) | (n = 40) |
| Absence d'endomètre | 5 | 10 | 3 | 3 |
| | (15,6) | (30,3) | (8,8) | (7,5) |
| Endomètre atrophique | 19 | 10 | 8 | 3 |
| | (59,4) | (30,3) | (23,5) | (7,5) |
| Endomètre sécrétoire | 8 | 12 | 22 | 34 |
| | (25,0) | (36,4) | (64,7) | (85,0) |
| Polype | 0 | 1 | 1(2,9) | 0 |
| | | (3,0) | | |

| | | | | |
|---|---|---|---|---|
| ( ) = pourcentage Aucun endomètre n'était prolifératif ou hyperplasique | | | | |

## Revendications

1. Méthode contraceptive chez la femme en âge de procréer, qui comprend l'administration quotidienne d'une composition contenant une dose d'acétate de nomégestrol s'échelonnant de 0,625 mg à 1,25 mg, et d'une dose d'estradiol ou d'un ester de celui-ci, exprimée en estradiol, s'échelonnant de 0,5 mg à 2 mg, pendant 21 à 28 jours, le rapport pondéral de la dose d'estradiol sur la dose d'acétate de nomégestrol s'échelonnant de 0,5 à 5, de préférence de 1 à 3.

2. Méthode selon la revendication 1, dans laquelle la composition contient 0,625 mg d'acétate de nomégestrol et 0,5 mg d'estradiol.

3. Méthode selon la revendication 1, dans laquelle la composition contient 0,625 mg d'acétate de nomégestrol et 1 mg d'estradiol.

4. Méthode selon la revendication 1, dans laquelle la composition contient 0,625 mg d'acétate de nomégestrol et 1,5 mg d'estradiol.

5. Méthode selon la revendication 1, dans laquelle la composition contient 0,625 mg d'acétate de nomégestrol et 2 mg d'estradiol.

6. Méthode selon l'une des revendications 1 à 5, dans laquelle la composition contient également un véhicule ou un diluant qui convient pour l'administration par voie digestive.

7. Méthode selon l'une des revendications 1 à 6, dans laquelle la composition est sous forme de comprimés nus ou pelliculés, de dragées, de gélules, de capsules, de pilules, de cachets ou de poudres.

8. Utilisation d'une composition contenant une dose d'acétate de nomégestrol s'échelonnant de 0,625 mg à 1,25 mg, et une dose d'estradiol ou d'un ester de celui-ci, exprimée en estradiol, s'échelonnant de 0,5 mg à 2 mg, comme agent contraceptif, le rapport pondéral de la dose d'estradiol sur la dose d'acétate de nomégestrol s'échelonnant de 0,5 à 5, de préférence de 1 à 3.

9. Utilisation selon la revendication 8, dans laquelle la composition contient 0,625 mg d'acétate de nomégestrol et 0,5 mg d'estradiol.

10. Utilisation selon la revendication 8, dans laquelle la composition contient 0,625 mg d'acétate de nomégestrol et 1 mg d'estradiol.

11. Utilisation selon la revendication 8, dans laquelle la composition contient 0,625 mg d'acétate de nomégestrol et 1,5 mg d'estradiol.

12. Utilisation selon la revendication 8, dans laquelle la composition contient 0,625 mg d'acétate de nomégestrol et 2 mg d'estradiol.

13. Article de conditionnement comprenant de 21 à 28 comprimés contenant une dose d'acétate de nomégestrol s'échelonnant de 0,625 à 1,25 mg, et une dose d'estradiol ou d'un ester de celui-ci, exprimée en estradiol, s'échelonnant de 0,5 mg à 2 mg, le rapport pondéral de la dose d'estradiol sur la dose d'acétate de nomégestrol s'échelonnant de 0,5 à 5, de préférence de 1 à 3.

14. Article de conditionnement selon la revendication 13, qui comprend en outre de 0 à 7 comprimés contenant un placébo.

## Patentansprüche

1. Methode zur Empfängnisverhütung bei der Frau im gebärfähigen Alter, die die tägliche Einnahme einer Zusammensetzung, die eine Dosis von Nomegestrolacetat in einer Menge von 0,625 mg bis 1,25 mg, und eine Dosis von Estradiol oder eines Esters desselben, ausgedrückt in Estradiol, in einer Menge von 0,5 mg bis 2 mg enthält, während 21 bis 28 Tagen umfasst, wobei das Gewichtsverhältnis der Dosis von Estradiol zur Dosis von Nomegestrolacetat 0,5 bis 5, vorzugsweise 1 bis 3, beträgt.

2. Methode nach Anspruch 1, bei der die Zusammensetzung 0,625 mg Nomegestrolacetat und 0,5 mg Estradiol enthält.

3. Methode nach Anspruch 1, bei der die Zusammensetzung 0,625 mg Nomegestrolacetat und 1 mg Estradiol enthält.

4. Methode nach Anspruch 1, bei der die Zusammensetzung 0,625 mg Nomegestrolacetat und 1,5 mg Estradiol enthält.

5. Methode nach Anspruch 1, bei der die Zusammensetzung 0,625 mg Nomegestrolacetat und 2 mg Estradiol enthält.

6. Methode nach einem der Ansprüche 1 bis 5, bei der die Zusammensetzung auch einen Trägerstoff oder ein Verdünnungsmittel enthält, die für die Einnahme auf enteralem Weg geeignet sind.

7. Methode nach einem der Ansprüche 1 bis 6, bei der die Zusammensetzung in Form von reinen Tabletten oder Filmtabletten, Dragees, Gelkapseln, Kapseln, Pillen, Oblatenkapseln oder Pulver vorhanden ist.

8. Verwendung einer Zusammensetzung, die eine Dosis von Nomegestrolacetat in einer Menge von 0,625 mg bis 1,25 mg, und eine Dosis von Estradiol oder eines Esters desselben, ausgedrückt in Estradiol, in einer Menge von 0,5 mg bis 2 mg als Empfängnis verhütenden Wirkstoff enthält, wobei das Gewichtsverhältnis der Dosis von Estradiol zur Dosis von Nomegestrolacetat 0,5 bis 5, vorzugsweise 1 bis 3, beträgt.

9. Verwendung nach Anspruch 8, bei der die Zusammensetzung 0,625 mg Nomegestrolacetat und 0,5 mg Estradiol enthält.

10. Verwendung nach Anspruch 8, bei der die Zusammensetzung 0,625 mg Nomegestrolacetat und 1 mg Estradiol enthält.

11. Verwendung nach Anspruch 8, bei der die Zusammensetzung 0,625 mg Nomegestrolacetat und 1,5 mg Estradiol enthält.

12. Verwendung nach Anspruch 8, bei der die Zusammensetzung 0,625 mg Nomegestrolacetat und 2 mg Estradiol enthält.

13. Verpackungsartikel, umfassend 21 bis 28 Tabletten, die eine Dosis von Nomegestrolacetat in einer Menge von 0,625 mg bis 1,25 mg, und eine Dosis von Estradiol oder eines Esters desselben, ausgedrückt in Estradiol, in einer Menge von 0,5 mg bis 2 mg enthalten, wobei das Gewichtsverhältnis der Dosis von Estradiol zur Dosis von Nomegestrolacetat 0,5 bis 5, vorzugsweise 1 bis 3, beträgt.

14. Verpackungsartikel nach Anspruch 13, der ferner 0 bis 7 Tabletten, die ein Plazebo enthalten, umfasst.

## Claims

1. Contraceptive method for women of child-bearing age, comprising the daily administration of a composition containing an amount of nomegestrol acetate ranging from 0.625 mg to 1.25 mg and an amount of estradiol or an ester thereof, expressed in estradiol, ranging from 0.5 mg to 2 mg, for a period of 21 to 28 days, wherein the weight ratio of the amount of estradiol to the amount of nomegestrol acetate is in the range from 0.5 to 5, preferably from 1 to 3.

2. The method according to claim 1, wherein the composition contains 0.625 mg nomegestrol acetate and 0.5 mg estradiol.

3. The method according to claim 1, wherein the composition contains 0.625 mg nomegestrol acetate and 1 mg estradiol.

4. The method according to one of claim 1 to 3, wherein the composition contains 0.625 mg nomegestrol acetate and 1.5 mg estradiol.

5. The method according to claim 1, wherein the composition contains 0.625 mg nomegestrol acetate and 2 mg estradiol.

6. The method according to one of claims 1 to 5, wherein the composition also contains a vehicle or diluent suitable for administration via the digestive route.

7. The method according to one of claims 1 to 6, wherein the composition is in the form of plain or film-coated tablets, sugar-coated tablets, soft gelatin capsules, wafer capsules, pills, cachets or powders.

8. Use of a composition containing an amount of nomegestrol acetate ranging from 0.625 mg to 1.25 mg and an amount of estradiol or an ester thereof, expressed in estradiol, ranging from 0.5 mg to 2 mg, as a contraceptive agent, wherein the weight ratio of the amount of estradiol to the amount of nomegestrol acetate is in the range from 0.5 to 5, preferably from 1 to 3.

9. The use according claim 8, wherein the composition contains 0.625 mg nomegestrol acetate and 0.5 mg estradiol.

10. The use according to claim 8, wherein the composition contains 0.625 mg nomegestrol acetate and 1 mg estradiol.

11. The use according to claim 8, wherein the composition contains 0.625 mg nomegestrol acetate and 1.5 mg estradiol.

12. The use according to claim 8, wherein the composition contains 0.625 mg nomegestrol acetate and 2 mg estradiol.

13. A packaging article comprising from 21 to 28 tablets containing an amount of nomegestrol acetate ranging from 0.625 mg to 1.25 mg and an amount of estradiol or an ester thereof, expressed in estradiol, ranging from 0.5 mg to 2 mg, wherein the weight ratio of the amount of estradiol to the amount of nomegestrol acetate is in the range from 0.5 to 5, preferably from 1 to 3.

14. The packaging article according to claim 17, further comprising 0 to 7 tablets containing a placebo.
